# EUROPEAN PATENT SPECIFICATION

(11) **EP 1 377 315 B1**
(45) Date of publication and mention of the grant of the patent: **03.01.2007**
(21) Application number: 02735219.4
(22) Date of filing: 03.04.2002
(51) Int. Cl.: A61K 47/38, A61K 31/436

(54) **PHARMACEUTICAL COMPOSITIONS OF RAPMYCINS**
PHARMAZEUTISCHE ZUSAMMENSETZUNGEN VON RAPAMYCINES
COMPOSITIONS PHARMACEUTIQUES DE RAPAMYCINES

(30) Priority: 04.04.2001 GB 0108498
(43) Date of publication of application: 07.01.2004
(73) Proprietor: Novartis AG, 4056 Basel (CH); Novartis Pharma GmbH, 1230 Wien (AT)
(72) Inventor: LEDERGERBER, Dorothea, 79594 Inzlingen (DE)
(86) International application number: PCT/EP2002/003694
(87) International publication number: WO 2002/080978

(56) References cited:
- EP-A- 0 240 773
- EP-A- 0 406 791
- EP-A- 1 074 255
- WO-A-00/07594
- WO-A-96/13249
- WO-A-02/096419
- US-A- 5 368 865

## Description

The invention relates to topical compositions comprising ascomycins for the treatment of skin disorders.

Ascomycins have a variety of useful pharmacological actions, e.g. immunosuppression, and may be administered topically. However, inter alia in view of their physicochemical properties, e.g. high molecular weight and lipophilicity, ascomycins have posed problems for topical administration and formulation development.

A large number of derivatives, antagonists, agonists and analogues of FK506, which retain the basic structure and at least one of the biological properties (for example immunological properties) of FK506, are now known. These compounds are described in a large number of publications, e.g. EP 184162, EP 315978, EP 323042, EP 423714, EP 427680, EP 465426, EP 474126, WO 91/13889, WO 91/19495, EP 484936, EP 532088, EP 532089, EP 569337, EP 626385, WO 93/5059 and the like.

The active agent may be an ascomycin or a derivative thereof, e.g. a compound of the FK506 class. FK506 is a known macrolide that is produced by Streptomyces tsukubaensis No. 9993. It is a potent immunosuppressant. The structure of FK506 is given in the Merck Index, 12th Edition, Entry no. 9200. Methods of preparing FK506 are described in EP 184162. Ascomycins and derivatives thereof are referred to hereafter as "ascomycins".

A preferred ascomycin is disclosed in EP 427680, as Example 66a, also named 33-epi-chloro-33-desoxy-ascomycin (hereinafter referred to as "Compound A"). Other preferred ascomycins are disclosed in EP 465426, EP 569337, and in EP 626385, e.g. as Example 6d in EP 569337 (hereinafter "Compound B") or as Example 8 in EP 626385 (hereinafter "Compound C").

Water-free, oil-based ointments containing an ascomycin and solubilizing and adsorption promoting agents to dissolve the compound are disclosed in EP 474126. Compositions containing water have been described in e.g. EP 484936 in the form of fine suspensions or in WO 96/13249 in the form of emulsions.

Preparations comprising an ascomycine and a cellulose derivative, e.g. HPMC, are known in the state of the art, see e.g. WO-A-007594, EP-A-406791, US-A-5368865, EP-A-240773, EP-A-1074255, WO-A-9613249 and WO-A-02964419.

It has been observed that ascomycins may crystallise in compositions comprising an ascomycin e.g. in dissolved form, e.g. dissolved in topical compositions, after several months of storage, e.g. of 3 to 36 months, e.g. of 4 to 24 months. After exhaustive testing, it has now been found that pharmaceutically acceptable cellulose derivatives inhibit or reduce crystal growth of ascomycins, e.g. in dissolved form, in pharmaceutical compositions.

Suitable cellulose derivatives include e.g. hydroxypropyl methyl cellulose (HPMC), ethyl cellulose (EC), hydroxyethyl cellulose (HEC), hydroxyethyl ethyl cellulose (HEEC), hydroxypropyl cellulose (HPC), methyl cellulose (MC) and mixtures thereof, preferably hydroxypropyl methyl cellulose.

Accordingly, the invention provides for a pharmaceutical composition, e.g. a topical composition, e.g. in the form of an emulsion, comprising an ascomycin, e.g. in dissolved form, and a pharmaceutically acceptable cellulose derivative for inhibiting or reducing crystal growth.

In another aspect, the present invention provides for the use of a pharmaceutically acceptable cellulose derivative to inhibit or reduce crystal growth of ascomycins in pharmaceutical compositions, e.g. in topical compositions, comprising an ascomycin, e.g. in dissolved or suspended form.

In a further aspect, the invention provides a method of preventing or reducing crystal growth of ascomycins in pharmaceutical compositions comprising an ascomycin in dissolved form, e.g. dissolved in topical compositions, by admixing a pharmaceutically acceptable cellulose derivative thereto.

The active agent is present in the compositions of the present invention e.g. in an amount of from 0.05 % to 3 % by weight, e.g. from 0.1 % to 2 % by weight, e.g. from 0.6 % to 1.2 %, e.g. from 0.4 % to 1 % by weight, based on the total weight of the composition.

The pharmaceutically acceptable cellulose derivatives may be present in an amount of e.g. from about 0.1% to about 5 % %, e.g. 1 % to 2 % by weight, based on the total weight of the composition, depending on the nature of the cellulose derivative.

Preferably, the ascomycin is in dissolved or suspended form, e.g. dissolved in a topical composition. The composition according to the present invention preferably is in the form of an emulsion; e.g. a cream or an ointment.

Accordingly, in a further aspect the invention provides a pharmaceutical composition in the form of an emulsion comprising an ascomycin in dissolved form and a pharmaceutically acceptable cellulose derivative. The emulsion may be an oil-in-water emulsion or a water-in-oil emulsion. The compositions are preferably oil-in-water emulsions, e.g. in the form of a cream or in the form of an emulsion gel.

The pharmaceutical compositions in form of an emulsion according to this aspect of the invention therefore further comprise pharmaceutically acceptable hydrophilic and lipophilic components.

Hydrophilic components suitable for an emulsion include e.g.:
i) alkanediols such as propyleneglycol (1,2-propanediol), butyleneglycol; 2-ethyll,3-hexanediol, hexyleneglycol (2-methyl-2,4-pentanediol) and the like, preferably of up to 8 carbon atoms;
ii) ether diols, such as dipropyleneglycol, diethylenegylcol and the like, preferably of up to 8 carbon atoms;
iii) diether alcohols, such as diethyleneglycol monoethylether and the like; and/or
iv) water, e.g. sterilized water.

The hydrophilic components i) to iii) are preferably present in an amount of about 5 % to about 50 % by weight, based on the total weight of the composition, more preferably about 5 % to about 20 % and even more preferably about 5 % to about 10 %. Water may be present in an amount of about 20 % to about 80 %, more preferably about 25 % to about 75 % and even more preferably about 35 % to about 65 % by weight, based on the total weight of the composition.

Lipophilic components suitable for an emulsion include e.g.:
i) saturated or unsaturated fatty alcohols, such as a C₁₂ to C₁₈, e.g. C₁₆ to C₁₈ fatty alcohols, preferably unsaturated fatty alcohols, preferably oleyl or elaidic alcohol, preferably in an amount of about 2 % to about 15 % and even more preferably of about 5 % to about 10 % by weight, based on the total weight of the composition;
ii) liquid oils, e.g. medium chain triglycerides obtained from fractionated vegetable oils, such as capryl/caprinic acid triglycerides such as a triglyceride commercially available under the trademark Miglyol® 812, having a molecular weight of about 520 Da, n_{D}²⁰ of about 1.448 to 1.450 and viscosity of 0.28 to 0.32 Pa's, in an amount of about 5 % to about 60 % by weight, based on the total weight of the emulsion, and preferably about 5 % to about 15 %;
iii) thickening agents, such as solid alcohols, having e.g. a C₁₂ to C₂₄ chain, e.g. cetyl and stearyl alcohol, hydrogenated castor oil, e.g. Cutina® HR, yellow wax, white wax, cetyl ester wax, microcrystalline wax and the like, in an amount of about 2 % to about 30 % by weight based on the total weight of the emulsion and more preferably about 2 % to about 10 %, e.g. about 8 %;
iv) lipophilic bases, such as natural wax, petroleum jelly, e.g. Vaseline®, e.g. Petrolatum@, thick paraffin, wool wax alcohols, e.g. Eucerinum® or Eucerin®, wool wax derivatives, triglyceride waxes, e.g. Softisan® 378 or Plastibase®, and the like, in an amount of about 5 % to about 40 %; and/or
v) mixtures of any of components i) to iv).

The lipophilic components may be present in about 20 % to about 80 %, more preferably about 25 % to about 75 % and even more preferably about 35 % to 65 % by weight, based on the total weight of the composition.

The compositions in form of an emulsion according to the present invention may further comprise emulsifiers. Such emulsifiers are described in standard texts such as Fiedler, H.P., Lexikon der Hilfsstoffe für Pharmazie, Kosmetik und angrenzende Gebiete (1996), Editio Cantor Verlag Aulendorf (Germany), and Kibbe, A.H., Handbook of Pharmaceutical Excipients (2000), a joint publication of Pharmaceutical Press, London (UK) and American Pharmaceutical Association, Washington (US). Examples of suitable emulsifiers include:
i) propyleneglycol mono- and di- fatty acid esters such as propyleneglycol caprylate, commercially available under the trademark Miglyol® 840 (Fiedler, loc. cit., p. 1009), propyleneglycol dilaurate, propyleneglycol hydroxystearate, propyleneglycol isostearate, propyleneglycol laurate, propyleneglycolrinoleate, and propyleneglycol stearate;
ii) polyoxyethylene-sorbitan-fatty acid esters, for example mono- and tri-lauryl, palmityl, stearyl and oleyl esters of the type known and commercially available under the trademark Tween® (Fiedler, loc. cit. p.1615 ff), including the products Tween®
   20 [polyoxyethylene(20)sorbitanmonolaurate],
   21 [polyoxyethylene(4)sorbitanmonolaurate],
   40 [polyoxyethylene(20)sorbitanmonopalmitate],
   60 [polyoxyethylene(20)sorbitanmonostearate],
   65 [polyoxyethylene(20)sorbitantristearate],
   80 [polyoxyethylene(20)sorbitanmonooleate],
   81 [polyoxyethylene(5)sorbitanmonooleate],
   85 [polyoxyethylene(20)sorbitantrioleate];
   especially preferred products of this class are Tween® 60 and Tween® 65;
iii) polyoxyethylene fatty acid esters, for example polyoxyethylene stearic acid esters of the type' known and commercially available under the trademark Myrj® (Fiedler, loc. cit., p.1042; Handbook of Pharmaceutical Excipients, loc. cit., page 420). An especially preferred product of this class is e.g. Myrj® 52, a polyoxyethylene 40 stearate having D²⁵ of about 1.1, a melting point of about 40 to 44°C, a HLB value of about 16.9, an acid value of about 0 to 1 and a saponification number of about 25 to 35;
iv) polyoxyethylene-polyoxypropylene copolymers and block co-polymers, such as those known and commercially available under the trademarks Pluronic®, Emkalyx® and Poloxamer® (Fiedler, loc. cit., p. 1200, 1203; Handbook of Pharmaceutical Excipients, loco cit., page 386) and in particular Poloxamer® 188 and Pluronic® F68, having a D²⁵ of about 1.1, a melting point of about 40 to 44°C, and a HLB value of about 16.9;
v) dioctylsulfosuccinate or di-[2-ethylhexyl]-succinate;
vi) phospholipids, in particular lecithins (Fiedler, loc. cit., p. 910, 1184). Lecithins suitable for use in the compositions of the invention include egg lecithins or soybean lecithins, in particular soybean lecithins, e.g. as known and commercially available under the trademark Phospholipon® 80 from Rhone Poulenc Rorer. Phospholipon® 80 is a phospholipid fraction with about 76 % phosphatidylcholine, about 8 % phosphatidic acid, about 4% phosphatidyl ethanolamine, and about 9% other lipids (manufacturer information);
vii) salts of fatty alcohol sulfates, such as sodium lauryl sulfate, and sodium cetyl stearyl sulfate, known and commercially available e.g. under the trademark Lanette® E (Fiedler, loc. cit., 2, p. 892);
viii) sorbitan fatty acid esters, e.g. sorbitan mono C₁₂₋₁₈ fatty acid esters, or sorbitan tri C₁₂₋₁₈ fatty acid esters, known and commercially available under the trademark Span® or Arlacel®. Particularly preferred are the products Arlacel® 60, a Sorbitan monostearate, having a HLB of about 4.7, a melting point of about 53°C (Fiedler, loc. cit., p. 192; Handbook of Pharmaceutical Excipients, loc. cit., page 511) or Span® 80, a Sorbitan monooleate, having a D²⁵ of about 1, a HLB of about 4.3, a viscosity of about 950 to 1100 cP (Fiedler, loc. cit., 2, p. 1430; Handbook of Pharmaceutical Excipients, loc. cit., page 511);
ix) glycerine monostearate, known and commercially available under the trademark Imwitor® (Fiedler, loc. cit., p. 799; Handbook of Pharmaceutical Excipients, loc. cit., page 225), particularly Imwitor 960;
x) esters of polyethylene-glycol glycerol ethers that have at least one free hydroxyl group and aliphatic C₆-C₂₂ carboxylic acids moieties. Examples include PEG-20 glycerine monostearate;
xi) reaction products of a natural or hydrogenated castor oil and ethylene oxide. Polyethyleneglycol hydrogenated castor oils are available under the trademark Cremophor® (Fiedler, loc. cit., p. 392). Particularly suitable are Cremophor® RH 40, having a saponification value of about 50 to 60, an acid value less than about 1, a water content (Fischer) less than about 2 %, a n_{D}⁶⁰ of about 1.453 to 1.457 and a HLB of about 14 to 16; Cremophor® RH 60, having a saponification value of about 40 to 50, an acid value less than about 1, an iodine value of less than about 1, a water content (Fischer) of about 4.5 to 5.5 %, a n_{D}²⁵ of about 1.453 to 1.457 and a HLB of about 15 to 17, and Cremophor® EL, having a molecular weight (by steam osmometry) of about 1630, a saponification value of about 65 to 70, an acid value of about 2, an iodine value of about 28 to 32 and a n_{D}²⁵ of about 1.471. Also suitable are various tensides available under the trademarks Nikkol® (Fiedler, loc. cit., p. 1087), Emulgin® (Fiedler, loc. cit., p. 545), Mapeg® (Fiedler, loc. cit., p. 967) and Incrocas® (Fiedler, loc. cit., p. 800);
xii) stearic acid;
xiii) oil and wax based emulsifiers such as cetyl alcohol and emulsifying wax;
xiv) polyoxyethylene glyceride as commercially available under the trademark Labrafil® (Fiedler, loc. cit., p. 880), particularly Labrafil® M2130 CS;
xv) polyoxyethylene alkyl ethers, e.g. polyoxyethylene glycol ethers of C₁₂ to C₁₈ alcohols, e.g. polyoxyethylene cetyl ether or polyoxyethylene oleyl ether, or polyoxyethylene stearyl ether, as known and commercially available under the trademark Brij® (Fiedler, loc. cit., p. 259; Handbook of Pharmaceutical Excipients, loc, cit., page 407);
xvi) glycerine sorbitan fatty acid esters as commercially available under the trademark Arlacel ® 481 (Fiedler, loco cit., p. 192; molecular weight of about 630, HLB value of about 4.5);
xvii) sucrose esters, e.g. sucrose fatty acid esters. The fatty acid moiety may comprise saturated or unsaturated fatty acids or mixtures thereof. Particularly suitable are C₆₋₁₈-fatty acid saccharide mono- or diesters, in particular water-soluble C₆₋₁₈ fatty acid saccharide mono- or diesters. Especially suitable are caproic (C₆), caprylic (C₈), capric (C₁₀), lauric (C₁₂), myristic (C₁₄), palmitic (C₁₆), oleic (C₁₈), ricinoleic (C₁₈) and 12-hydroxystearic (C₁₈) acid saccharide mono- or diesters, e.g. sucrose distearate, e.g. as known and commercially available under the trademark Sucro Ester® 7 from Gattefossé, France; and
xviii) mixtures of any of components i) to xvii).

It is to be appreciated that emulsifiers may be complex mixtures containing side products or unreacted starting products involved in the preparation thereof, e.g. emulsifiers made by polyoxyethylation may contain another side product, e.g. polyethylene glycol.

If the emulsion is an oil-in-water emulsion, the emulsifier selected preferably has a HLB value of 10 to 15. If the emulsion is a water-in-oil emulsion, the emulsifier selected has a HLB value of 4 to 8. Preferably the emulsifiers are present in an amount of about 1 % to about 30 % by weight, based on the total weight of the composition, and more preferably about 1.0 % to about 2.5 %.

Further components, e.g. preserving agents, such as benzyl alcohol, sorbic acid, chlorocresol, propyl p-hydroxybenzoate and methyl p-hydroxybenzoate, and antioxidants, such as butyl-hydroxytoluene, ascorbyl palmitate, sodium pyrosulfite, butyl hydroxy anisole and tocopherol may be included as appropriate. Preserving agents and antioxidants are preferably present in an amount of about 0.01 % to about 2.5 %, e.g. about 1 % by weight based on the total weight of the composition.

If desired, pH modifying agents may be included to bring the pH of the composition to between about 4 and 6, or by adding a pharmaceutically acceptable buffer system of a pH of between about 4 and 6.

In another aspect the invention provides a composition as defined above for use in the treatment of inflammatory or hyperproliferative skin diseases or of cutaneous manifestations of immunologically-mediated diseases.

It further provides a method for treating inflammatory or hyperproliferative skin diseases or cutaneous manifestations of immunologically-mediated diseases comprising administering a composition as defined above to the skin of a patient in need thereof.

It further provides the use of a composition as defined above in the preparation of a medicament for the treatment of inflammatory or hyperproliferative skin diseases or of cutaneous manifestations of immunologically-mediated diseases.

The compositions of the invention in form of emulsion may be prepared in a conventional manner by working up the components into a pharmaceutical composition.

For example, the composition of the invention in form of emulsion may be obtained by dissolving the ascomycin in the lipophilic components. Optionally, the resulting mixture may be warmed, to e.g. about 60°C and optionally cooled to room temperature afterwards. The mixture is then emulsified with the aqueous components at the same temperature containing the pharmaceutically acceptable cellulose derivative, and, if necessary, suitable emulsifiers. Other excipients may be added to the appropriate phase as is conventional. Alternatively, the pharmaceutically acceptable cellulose derivative may be dispersed in the lipophilic components.

The compositions of the invention are useful in the treatment of inflammatory or hyperproliferative skin diseases or of cutaneous manifestations of immunologically-mediated diseases. Examples of such diseases are psoriasis, atopic dermatitis, contact dermatitis and further eczematous dermatitises, seborrhoeic dermatitis. Lichen planus, Pemphigus, bullous Pemphigoid, Epidermolysis bullosa, urticaria, angioedemas, vasculitides, erythemas, cutaneous eosinophilias, Lupus erythematous and Alopecia areata.

The utility of the topical compositions can be observed in standard clinical test using concentration of ascomycin of 0.01 % to 10% by weight, based on the total weight of the composition, preferably 0.1 % to 1%. The utility can also be observed using standard animal models as described in EP 315978.

The exact amount of ascomycin and of composition to be administered depends on several factors, for example the desired duration of treatment and the rate of release of the ascomycin. Satisfactory results are obtained in larger mammals, e.g. humans, with local application over the area to be treated of a 0.01 % to 10 %, preferably 0.1 % to 2 %, more preferably 1 %, by weight, concentration of the ascomycin once or several times a day (e.g. 1, 2 to 5 times a day). In general the compositions may be applied to areas of skin as small as 1 cm² to as large as 1 m². Suitable skin loadings of the ascomycin fall within the range of 0.1 mg/cm² to 2 mg/cm².

The compositions of this invention are straightforward to prepare and are well-tolerated on human skin. Good skin penetration and permeation rates may be achieved using the compositions of this invention.

Following is a description by way of example of compositions of the invention:

### Examples 1 to 3

A cream is prepared as described in Table 1 (amounts in g).

The compositions of Examples 1 to 3 are found to be stable. After 2 months of storage at cycling temperatures (-5°C/40°C) or after 12 months of storage at 25°C, the compositions are found to be substantially free of crystals (crystal count less than 50) as judged by their appearance using polarized light microscopy.

Compound A may be replaced with Compound B or Compound C.

**Table 1**

| | Ex.1 | Ex.2 | Ex.3 |
|---|---|---|---|
| Compound A | 1.0 | 1.0 | 1.0 |
| hydroxypropylmethyl cellulose | 1.0 | 0.5 | 1.0 |
| oleyl alcohol | 10.0 | 10.0 | 10.0 |
| triglycerides, medium chained | 15.0 | 15.0 | 15.0 |
| glycerol monostearate | 2.0 | 2.0 | 2.0 |
| sorbitan monostearate | - | - | 3.0 |
| polyoxyethylene(60)sorbitanmonolaurate | - | - | 5.0 |
| cetyl alcohol | 4.0 | 4.0 | 4.0 |
| stearyl alcohol | 4.0 | 4.0 | 4.0 |
| sodium cetylstearyl sulfate | 1.0 | 1.0 | - |
| benzyl alcohol | 1.0 | 1.0 | - |
| methyl paraben | - | - | 0.07 |
| propyl paraben | - | - | 0.03 |
| citric acid | 0.05 | 0.05 | 0.05 |
| sodium hydroxide | 0.02 | 0.02 | 0.02 |
| propylene glycol | 5.0 | 5.0 | 5.0 |
| water, purified | to 100 | to 100 | to 100 |

### Examples 4 to 6 and comparative example 7

A cream is prepared as described in Table 2 (amounts in g).

After 0 months, and 4 months of storage at cycling temperatures (-5°C/40°C), the compositions of example 4 to 6 and of comparative example 7 were examined by polarized light microscopy at a magnification of 200. The crystals present were counted in ten randomly chosen fields. The number of crystals was visually assessed. The result of the crystal count is given in Table 3 (amounts in number of crystals).

**Table 2:**

| | Ex.4 | Ex.5 | Ex.6 | Ex.7 |
|---|---|---|---|---|
| Compound A | 1.5 | 1.5 | 1.5 | 1.5 |
| hydroxypropylmethyl cellulose | 0.1 | 0.5 | 1.0 | 0.0 |
| oleyl alcohol | 10.0 | 10.0 | 10.0 | 10.0 |
| triglycerides, medium chained | 15.0 | 15.0 | 15.0 | 15.0 |
| glycerol monostearate | 2.0 | 2.0 | 2.0 | 2.0 |
| cetyl alcohol | 4.0 | 4.0 | 4.0 | 4.0 |
| stearyl alcohol | 4.0 | 4.0 | 4.0 | 4.0 |
| sodium cetylstearyl sulfate | 1.0 | 1.0 | 1.0 | 1.0 |
| benzyl alcohol | 1.0 | 1.0 | 1.0 | 1.0 |
| citric acid | 0.05 | 0.05 | 0.05 | 0.05 |
| sodium hydroxide | 0.02 | 0.02 | 0.02 | 0.02 |
| propylene glycol | 5.0 | 5.0 | 5.0 | 5.0 |
| water, purified | to 100 | to 100 | to 100 | to 100 |

**Table 3:**

| | Ex. 4 | Ex. 5 | Ex. 6 | Ex.7 |
|---|---|---|---|---|
| 0 weeks | 0 | 0 | 0 | 0 |
| 4 weeks | 110 | 33 | 9 | 265 |

## Claims

1. Use of a pharmaceutically acceptable cellulose derivative to inhibit or reduce crystal growth of an ascomycin in a pharmaceutical composition comprising an ascomycin in dissolved or suspended form.

2. Use according to claim 1 wherein the pharmaceutically acceptable cellulose derivative is hydroxypropyl methyl cellulose.

3. Use according to claim 1 or 2 wherein the pharmaceutically acceptable cellulose derivative is present in an amount of about 0,1% to about 5% by weight, based on the total weight of the composition.

4. Use according to any one of claims 1 to 3 wherein the ascomycin is 33-epichloro-33-desoxy-ascomycin.

5. A method of preventing or reducing crystal growth of an ascomycin in a pharmaceutical composition comprising an ascomycin in dissolved or suspended form by admixing thereto a pharmaceutically acceptable cellulose derivative for inhibiting or reducing post-dissolution or -suspension crystal growth.

## Patentansprüche

1. Verwendung eines pharmazeutisch akzeptablen Cellulosederivats zur Inhibition oder Reduktion von Kristallwachstum eines Ascomycins in einer pharmazeutischen Zusammensetzung, die ein Ascomycin in gelöster oder suspendierter Form umfasst.

2. Verwendung nach Anspruch 1, worin das pharmazeutisch akzeptable Cellulosederivat Hydroxypropylmethylcellulose ist.

3. Verwendung nach Anspruch 1 oder 2, worin das pharmazeutisch akzeptable Cellulosederivat in einer Menge von 0,1 Gew.-% bis etwa 5 Gew.-%, bezogen auf das Gesamtgewicht der Zusammensetzung, vorhanden ist.

4. Verwendung nach einem der Ansprüche 1 bis 3, worin das Ascomycin 33-Epichlor-33-desoxyascomycin ist.

5. Verfahren zur Prävention oder Reduktion von Kristallwachstum eines Ascomycins in einer pharmazeutischen Zusammensetzung, die ein Ascomycin in gelöster oder suspendierter Form umfasst, durch Vermischung dieser Zusammensetzung mit einem pharmazeutisch annehmbaren Cellulosederivat zur Inhibition oder Reduktion von Kristallwachstum post Auflösung oder Suspension.

## Revendications

1. Utilisation d'un dérivé de cellulose pharmaceutiquement acceptable destiné à inhiber ou à réduire la croissance cristalline d'une ascomycine dans une composition pharmaceutique comprenant une ascomycine sous forme dissoute ou suspendue.

2. Utilisation selon la revendication 1 **caractérisée en ce que** le dérivé de cellulose pharmaceutiquement acceptable est de l'hydroxypropyl méthylcellulose.

3. Utilisation selon la revendication 1 ou 2 **caractérisée en ce que** le dérivé de cellulose pharmaceutiquement acceptable est présent selon une quantité comprise dans la gamme allant d'environ 0,1% à environ 5% en poids sur base du poids total de la composition.

4. Utilisation selon l'une quelconque des revendications 1 à 3 **caractérisée en ce que** l'ascomycine est 33-épichloro-33-désoxy-ascomycine.

5. Méthode permettant de prévenir ou de réduire la croissance cristalline d'une ascomycine dans une composition pharmaceutique comprenant une ascomycine sous forme dissoute ou suspendue en la mélangeant au dérivé de cellulose pharmaceutiquement acceptable destiné à inhiber ou à réduire la croissance cristalline post-dissolution ou -suspension.
